# FASCICULE DE BREVET EUROPEEN

(11) **EP 2 209 425 B1**
(45) Date de publication et mention de la délivrance du brevet: **09.11.2016**
(21) Numéro de dépôt: 08856666.6
(22) Date de dépôt: 18.09.2008
(51) Int. Cl.: A61B 10/02, A61B 34/20, A61B 34/30, A61B 8/12

(54) **SYSTÈME ET PROCÉDÉ D'IMAGERIE ET DE LOCALISATION PONCTIONS SOUS ÉCHOGRAPHIE PROSTATIQUE**
SYSTEM UND VERFAHREN ZUR BILDGEBUNG UND ZUR LOKALISATION VON PROSTATA-PUNKTIONEN MITTELS ULTRASCHALL
SYSTEM AND METHOD FOR IMAGING AND LOCATING BIOPSIES UNDER PROSTATIC ECHOGRAPHY

(30) Priorité: 18.09.2007 FR 0706544
(43) Date de publication de la demande: 28.07.2010
(73) Titulaire: Koelis, 38100 Grenoble (FR); Université Grenoble Alpes, 38400 Saint-Martin-d'Hères (FR)
(72) Inventeur: LEROY, Antoine, F-38240 Meylan (FR); BAUMANN, Michael, F-38000 Grenoble (FR); MOZER, Pierre, F94300 Vincennes (FR); TROCCAZ, Jocelyne, F-38320 Eybens (FR); DAANEN, Vincent, F-38134 Saint Joseph De Riviere (FR)
(74) Mandataire: Novagraaf Technologies
(86) Numéro de dépôt international: PCT/FR2008/001306
(87) Numéro de publication internationale: WO 2009/071766

(56) Documents cités:
- EP-A- 1 804 079
- WO-A-2004/019799
- WO-A-2006/089426
- PHEE L ET AL: "Ultrasound Guided Robotic System for Transperineal Biopsy of the Prostate" ROBOTICS AND AUTOMATION, 2005. PROCEEDINGS OF THE 2005 IEEE INTERNATIO NAL CONFERENCE ON BARCELONA, SPAIN 18-22 APRIL 2005, PISCATAWAY, NJ, USA,IEEE, 18 avril 2005 (2005-04-18), pages 1315-1320, XP010871859 ISBN: 978-0-7803-8914-4

## Description

La présente invention concerne le domaine des équipements destinés au diagnostic et au traitement thérapeutique assistés par ordinateur, appliqués aux pathologies de la prostate, et notamment le cancer. Elle concerne plus précisément un système de localisation de prélèvements biologiques in vivo effectués à l'aide d'une sonde échographique transrectale.

### DOMAINE DE L'INVENTION

Les ponctions de prostate, notamment la ponction-biopsie, se déroulent de manière standard sous échographie transrectale. La sonde échographique porte un guide de ponction mécaniquement positionné de telle manière que la trajectoire de l'aiguille de ponction se trouve dans un/le plan d'acquisition sagittal de la sonde. Le positionnement de l'aiguille par rapport à l'organe se fait par guidage visuel notamment à l'aide des images échographiques dudit plan d'acquisition. Pour une sonde 3D, on entendra par « plan sagittal » le « champ de vision » de la sonde, et le cas échéant le plan médian du champ de vision.

Dans le cas de la ponction-biopsie, pour réaliser ses analyses le praticien se conforme à un schéma de ponction prédéfini, d'au moins six ponctions, douze étant la norme. À partir de ce schéma, il doit répartir les prélèvements de manière homogène dans la zone de la prostate, en regard de la tête de sonde rectale, pour maximiser les chances de détecter un éventuel foyer cancéreux.

La principale difficulté est d'assurer une répartition cohérente des points de ponction, qui soit optimale au regard des premiers résultats diagnostiques, sur un organe qui n'est pas directement visible par l'opérateur, qui n'est pas fixe et qui se déforme pendant l'intervention. La manipulation de la tête de la sonde requiert donc à la fois une grande dextérité et une forte expérience pour garantir que le point effectif du prélèvement correspond à celui du schéma de ponction planifié.

Cette difficulté à guider la tête de ponction vers une cible théorique implique a fortiori la méconnaissance par le praticien de l'endroit exact du prélèvement effectué. Ce manque d'information peut mener à un diagnostic imparfait et non utile aux séances de biopsies de répétition, ou à une thérapie non focalisée ou tout au moins non optimale.

L'information de localisation intra-prostatique de la tête de ponction est la clef d'un système de prise en charge efficace et global des pathologies de la prostate, et notamment le cancer.

### ART ANTÉRIEUR : demande internationale WO2005/110255

On a proposé dans la demande internationale WO2005/110255 une solution pour le ciblage et la biopsie de tissus effectués à des fins médicales, et en particulier un système de biopsie ciblée qui permet la planification des tissus à échantillonner, le ciblage de zones spécifiques de tissus en référence au plan, la capture de l'échantillon de tissus et l'enregistrement de l'emplacement source de l'échantillon de tissus. Le système est utilisé, en particulier, dans la collecte d'échantillons de tissus de la glande de la prostate. Il comprend une aiguille de biopsie flexible, un guide directionnel de ladite aiguille de biopsie, des moyens d'imagerie, un schéma de biopsie et des moyens d'enregistrement.

### INCONVENIENT DE CETTE SOLUTION WO2005/110255

Le problème de cette solution est que le repérage de la prostate sur les images est purement mécanique. De fait, l'image de l'aiguille par rapport à la prostate est calculée en faisant intervenir une succession de référentiels :
- le référentiel « salle d'opération », qui permet de déterminer la position de la sonde grâce au support ;
- le référentiel « corps du patient » qui est susceptible de bouger par rapport au référentiel « salle d'opération » ;
- le référentiel « organe » qui est susceptible de bouger et de se déformer par rapport au référentiel « corps du patient » ;
- le référentiel « sonde » qui est théoriquement fixe par rapport au référentiel « salle d'opération », mais les liaisons mécaniques ne sont pas absolument rigides et conduisent à un risque de décalage.

Le calcul de l'image de la sonde par rapport à la prostate se fait donc avec une accumulation - et plutôt une multiplication - des incertitudes, et conduit à des localisations de la sonde par rapport à la prostate avec une imprécision de plusieurs millimètres. Dès lors, la position des prélèvements par rapport à la glande ne peut être enregistrée ni exploitée.

Les conséquences sont importantes : la zone cancéreuse n'est connue qu'avec une précision de plusieurs millimètres, ce qui oblige à des traitements sur des zones inutilement larges. Dans ces conditions, un traitement thérapeutique, par exemple par irradiation locale, ne peut être focalisé avec précision sur le foyer cancéreux.

### ART ANTERIEUR : demande internationale WO2004/019799

On connaît également dans l'état de la technique la demande internationale WO2004/019799, qui est considéré l'état de la technique le plus proche de la présente invention, concernant une méthode pour déterminer la position d'une aiguille de biopsie dans un volume cible, ce dernier étant défini en tant qu'espace à l'intérieur du patient. Ce procédé consiste à :
- produire plusieurs images du volume cible,
- enregistrer spatialement les images, à produire une représentation tridimensionnelle du volume cible à partir des images enregistrées,
- déterminer l'emplacement de l'aiguille de biopsie dans la représentation tridimensionnelle du volume cible, et
- corréler l'emplacement de l'aiguille de biopsie déterminé avec les images enregistrées spatialement.

La présentation graphique de la représentation du volume cible comprend une description graphique de l'emplacement déterminé de l'aiguille de biopsie. Ce document décrit aussi une technique de localisation dans laquelle une caméra de suivi d'une cible de référence est fixée à une sonde ultrasonore, ce qui permet une localisation précise de la sonde dans un système de coordonnées.

Toutefois, dans la solution décrite dans ce document, l'espace à l'intérieur du patient n'est pas défini de manière précise : il peut être interprété en tant qu'ensemble de tissus appartenant au patient ou en tant que volume dont la position et l'orientation sont définies par rapport à un repère extérieur au patient.

Dans le premier cas, le système détermine la position de l'aiguille par rapport à l'anatomie du patient, tandis que dans le deuxième cas, le système détermine la position de l'aiguille par rapport à un repère extérieur au patient. Dans le deuxième cas, il est essentiel que le patient et l'organe ciblé ne bougent ni ne se déforment pas pendant l'intervention, sinon la position de l'aiguille calculée dans le volume cible ne correspond plus à la position de l'aiguille par rapport à l'anatomie du patient.

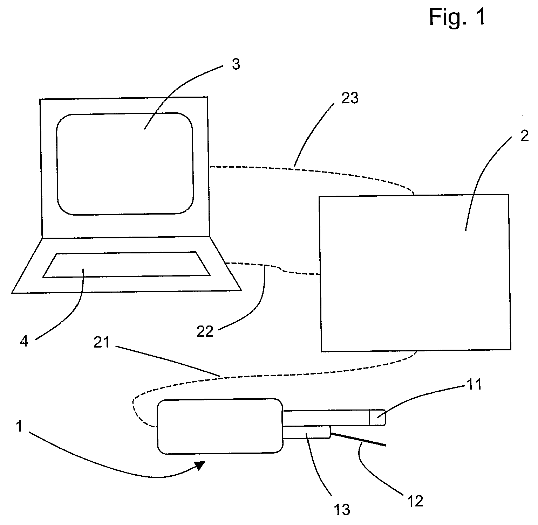

Il devient clair avec le volume englobant et le référentiel x-y-z dessinés dans la Fig.1 de cette demande internationale, ainsi qu'avec la description des méthodes de localisation (p.13 1.24 - p.14 1.35) que le système proposé se situe dans le deuxième cas. Plusieurs méthodes sont proposées, toutes visent à localiser la position de l'image échographique par rapport à un référentiel extérieur et donc non-anatomique. La première méthode (p.13, l. 24-37) est stéréotaxique et localise les images par rapport au repère de la caméra. La deuxième méthode est acoustique (p.13, 1.38-p.14, 1.4) et localise les images par rapport au repère de l'émetteur acoustique.

La troisième méthode proposée (p. 14, l. 5-1.35) est mécanique et consiste à fixer la trajectoire de l'aiguille via un guide par rapport à la sonde, et de fixer en même temps la prostate avec un harpon pour réduire sa mobilité, et de déterminer la position de la sonde (et donc de l'aiguille) avec un système mécanique (e.g. un bras articulé encodé) le repère de ce système. Le harpon permet de réduire les bougés de la prostate dus aux bougés de la sonde, mais il ne peut pas compenser les bougés du patient (qui est seulement localement anesthésié).

Pour résumer, le système proposé dans le document de l'art antérieur est capable de déterminer l'aiguille par rapport à un repère extérieur au patient et requiert donc que les tissus ciblés ne bougent pas pendant l'intervention pour obtenir des résultats cliniquement satisfaisants.

### INCONVENIENT DE CETTE SOLUTION WO2004/019799 ET SOLUTION APPORTEE PAR L'INVENTION

L'invention revendiquée est basée sur une méthode de localisation par rapport à un repère anatomique, ce dernier étant défini par une image de référence 3D qui contient l'anatomie ciblée, à savoir la prostate.

La mise en correspondance des tissus imagés dans les images de suivi et l'image de référence permet d'être indépendant de systèmes de localisation extérieurs tels que ceux décrits dans la demande internationale WO2004/019799. L'invention permet ainsi de gérer les « sauts » des patients bien connus par les praticiens, dus à des douleurs causées suite au déclenchement du pistolet de biopsie, grâce à l'utilisation d'un repère purement anatomique.

Les patients sautent environ dans 10 à 20% des interventions de biopsies de prostate transrectales. L'invention permet également d'éviter les problèmes liés aux bougés de la prostate causés notamment par les mouvements de la sonde endorectale.

Le document WO2004/019799 ne prévoit pas l'exploitation de l'une des images acquises pour déterminer une «image de référence » liée à la position initiale de la prostate.

La méthode proposée par ce document vise certes la même finalité, mais met en oeuvre un moyen totalement différent : elle propose d'équiper la partie externe de la sonde d'une caméra qui enregistre l'image d'un motif extérieur au patient, et servant au recalage de la position de la sonde.

La partie de la sonde qui ne pénètre pas dans le corps du patient est munie d'une caméra qui doit être dirigée vers un motif fixe, par exemple collé sur une paroi du bloc opératoire ou d'un équipement supportant le patient.

Les inconvénients de cette solution sont multiples :
- elle impose des contraintes gênant l'ergonomie et l'exploitation de la sonde,
- elle fournit un référentiel qui est extérieur, celui du bloc opératoire, et non pas directement lié à la zone d'observation à savoir la prostate elle-même,
- elle ne permet pas d'éviter les perturbations résultant déplacements intempestifs du patient
- elle implique le passage par une succession de référentiel dégradant la précision des transformations :
- Transformation « bloc opératoire/corps du patient »,
- Transformation « bloc opératoire / prostate ».

Ce document ne divulgue donc pas les caractéristiques revendiquées et ne les suggère pas d'une manière évidente pour l'homme du métier.

### ART ANTERIEUR : demande internationale WO2006/089426

Ce document a trait à un système et un procédé pour la réalisation d'une biopsie d'un volume cible et à un dispositif informatique pour sa planification. Un transducteur ultrasonique capture des données de volume ultrasonores à partir d'un volume cible. Un module d'enregistrement tridimensionnel enregistre les données de volume ultrasonores avec des données de volume supplémentaires associées au volume cible. Un module de planification de biopsie assure le traitement des données de volume ultrasonores et les données de volume supplémentaires en combinaison en vue de développer un plan de biopsie pour le volume cible. Une aiguille de biopsie réalise la biopsie du volume cible selon le plan de biopsie. Une solution consistant à injecter une image « supplémentaire » pré-opératoire, sur une image « sondage » per-opératoire servant à guider le geste du praticien (§46 p13). L'approche revendiquée dans la présente invention diffère en ce qu'elle vise la localisation de l'organe lors des ponctions, par rapport à une position de référence.

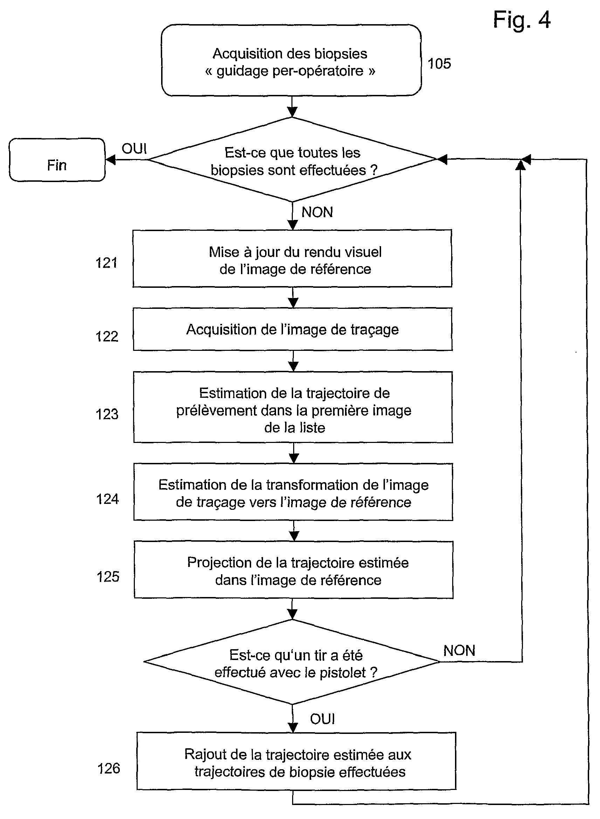

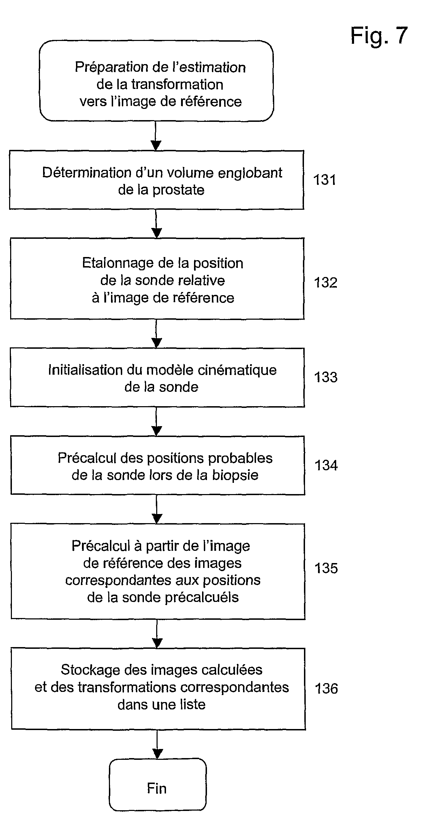

Le système décrit dans le document WO2006/089426 utilise également une localisation des images dans un référentiel externe et non-anatomique pour déterminer la position de l'aiguille dans le volume de référence (Fig. 4, 108 et p.22, 1.8-121 et §64 p16 avec le support et MCM). L'échographie « tri-dimensionnelle » reconstruite par ce procédé (Fig.7, Fig. 8, Fig4 132, §139 p11) diffère de l'échographie 3D vraie sur laquelle se base une variante avantageuse de la présente invention revendiquée, du point de vue de la quantité de données disponibles, des vitesses, cohérence et finesse de la reconstruction anatomique 3D.

Le système proposé dans le document WO2006/089426 est donc sujet aux mêmes défauts que ceux que nous identifiés pour le système décrit dans le document WO2004/019799. En particulier, les mouvements de l'organe et a fortiori du patient remettent en cause l'ensemble du procédé de guidage.

### OBJET DE L'INVENTION

Le but de l'invention est de remédier à cet inconvénient en proposant une solution permettant d'améliorer la précision des prélèvements biologiques et la localisation des carottes correspondantes.

De plus, la solution apportée par la présente invention permet :
- au niveau pré-opératoire de définir un planning de ponction, transférable au temps per-opératoire par recalage de données ;
- au niveau per-opératoire de guider l'aiguille et de visionner la distribution spatiale des ponctions déjà effectuées ;
- au niveau post-opératoire de visionner la distribution spatiale des ponctions effectuées ; dans le cas de la biopsie, il s'agit de la distribution des prélèvements et du tissu cancéreux identifié lors d'une analyse anatomopathologique, pour établir un diagnostic et aider à la planification d'un traitement focalisé.

Le guidage de l'aiguille per-opératoire et l'identification précise des sites de ponction, rendue possible par l'invention, rend un traitement post-opératoire focalisé possible.

À cet effet, l'invention concerne selon son acception la plus générale un système pour l'imagerie prostatique selon la revendication 1.

Dans un mode de réalisation particulier, le système est agencé de sorte à permettre une visualisation de la position de l'aiguille de prélèvement dans le référentiel « image de référence » ou le référentiel « image de suivi » (« tracking » en anglais) après ponction, en surimpression avec une image d'une partie au moins de la prostate présentant les différentes positions de prélèvements antérieurs.

Dans un autre mode de réalisation particulier, le système est agencé de sorte à permettre une visualisation en temps réel de la position, effectuée ou théorique, de l'aiguille de prélèvement dans le référentiel « image de référence» ou le référentiel « image de suivi », en surimpression avec une image d'une partie au moins de la prostate présentant les différentes positions de prélèvements antérieurs.

Dans un autre mode de réalisation particulier, le système est agencé de sorte à permettre une visualisation de la distribution du tissu cancéreux dans le référentiel « image de référence» après fusion de la distribution spatiale des ponctions marquées avec les résultats issus de l'analyse anatomopathologique.

De préférence, l'invention permet le recalcul d'une nouvelle image prostatique marquée lors de chaque nouveau prélèvement.

Selon une variante avantageuse, la fréquence de rafraîchissement des images acquises par la sonde échographique est d'au moins 3 images par seconde. Selon une alternative avantageuse, le système est agencé de sorte à permettre l'acquisition d'un flux d'image dont la périodicité est fonction de la fréquence de rafraîchissement des images échographiques, de la vitesse de transfert vers l'ordinateur et de la vitesse de traitement des images reçues.

Selon un premier exemple de mise en oeuvre, la sonde échographique est agencée de sorte à procéder à une imagerie tridimensionnelle.

Selon un deuxième exemple de mise en oeuvre, la sonde échographique est agencée de sorte à procéder à une imagerie de type « 2,5 dimensions » (images 2D localisées dans l'espace).

De préférence, le système selon l'invention est agencé de sorte à réaliser un marquage chronologique avec un numéro d'ordre mettant en oeuvre des moyens d'étiquetage univoque de chaque zone de prélèvement.

Selon un premier exemple de réalisation, les moyens d'étiquetage univoque sont générés manuellement, aiguille en place.

Selon un deuxième exemple de réalisation, les moyens d'étiquetage univoque sont générés par un déclenchement de l'acquisition de données manuel, ou automatique par la capture d'un signal sonore, lumineux, magnétique ou tactile, au moment précis du tir de biopsie.

Dans un mode particulier de réalisation, le système est agencé de sorte à mettre en oeuvre, en outre, un modèle cinématique dérivé des contraintes anatomiques spécifiques d'une ponction transrectale de la prostate, permettant d'identifier des positions probables de la sonde lors de l'intervention.

Dans un mode particulier de réalisation, le système est agencé de sorte à mettre en oeuvre, en outre, un processus de précalcul des positions probables de la sonde lors de la biopsie et des images correspondant aux positions de la sonde précalculées à partir de l'image de référence.

Avantageusement, le système est agencé de sorte à mettre en oeuvre, en outre, un algorithme de recalage d'images basé sur l'optimisation locale d'une mesure de similarité.

De préférence, le système comporte en outre un réceptacle pour le recueil des échantillons de tissu prélevés, avec un identifiant corrélé avec le marquage de la zone de prélèvement correspondant sur l'image unique de la prostate marquée. Avantageusement, le système embarque un système d'édition d'étiquettes à apposer sur le réceptacle, portant les identifiants uniques susmentionnés.Le système d'édition peut être constitué par une imprimante thermique ou une imprimante à jet d'encre, apposant un code-barre ou un code matriciel.

Selon un premier exemple de réalisation, la sonde rectale est une sonde à main sans positionnement robotisé.

Selon un deuxième exemple de réalisation, la sonde rectale est une sonde à main naviguée par un localisateur optique ou magnétique.

Selon un troisième exemple de réalisation, la sonde rectale est une sonde à positionnement robotisé.

### DESCRIPTION DÉTAILLÉE D'UN EXEMPLE DE RÉALISATION

L'invention sera mieux comprise à la lecture de la description qui suit, se rapportant à un exemple non limitatif de réalisation, où :
- la figure 1 est un schéma du système d'imagerie prostatique ;
- la figure 2 est un diagramme de procédure de gestion des biopsies de la prostate ;
- la figure 3 est un diagramme de procédure d'établissement du planning de biopsie ;
- la figure 4 est un diagramme d'une variante de procédé d'acquisition des biopsies, dit de « consultation per-opératoire » ;
- la figure 5 est un diagramme d'une variante de procédé d'acquisition des biopsies, dit de « guidage per-opératoire » ;
- la figure 6 est un diagramme d'une variante de procédé d'acquisition des biopsies, dit de « consultation post-opératoire » ;
- la figure 7 est un diagramme du procédé de préparation de l'estimation de transformations vers l'image de référence ;
- la figure 8A illustre le modèle cinématique de la sonde par rapport à la prostate dans une première position de ladite sonde;
- la figure 8B illustre le modèle cinématique de la sonde par rapport à la prostate dans une deuxième position de ladite sonde;
- la figure 8C illustre le modèle cinématique de la sonde par rapport à la prostate dans une troisième position de ladite sonde;
- la figure 9 est un diagramme du procédé d'estimation de l'image de suivi vers l'image de référence.

Le présent exemple de réalisation concerne un système mettant en oeuvre un procédé d'imagerie médicale permettant l'acquisition et l'analyse d'images échographiques 3D en vue de localiser des prélèvements ou aiguilles par rapport à la prostate. Un algorithme de recalage des images, par analyse statistique des similarités, permet une mise en correspondance de toutes les acquisitions dans un même référentiel lié à l'organe.

Au temps pré-opératoire, une interface graphique permet au clinicien de planifier un schéma de ponctions sur une première acquisition échographique. Plusieurs options lui sont proposées :
- un planning manuel sans contraintes ;
- un planning automatique équiréparti pour un nombre défini de prélèvements, avec possibilité de correction manuelle dans un second temps ;
- un planning différentiel, c'est-à-dire la visualisation des précédentes ponctions en superposition sur l'actuelle acquisition, en cas d'interventions répétées ;
- le transfert d'un planning effectué sur une imagerie externe par exemple de type IRM.

Le système peut être utilisé, et le procédé mis en oeuvre, selon trois modes :
- au temps per-opératoire, le système peut après chaque ponction représenter, sur une vue annexe à l'image échographique temps réel, la position des prélèvements effectués jusque-là au cours de l'intervention. Pour chaque ponction, l'acquisition échographique 3D pourra être soit manuelle, soit automatique par le biais d'un détecteur par exemple sonore. On parlera ci-après de « consultation per-opératoire » pour ce mode de réalisation.
- au même temps per-opératoire, si le matériel informatique le permet, le système peut représenter en temps réel sur l'image échographique de guidage ou sur un modèle, la position actuelle du guide de ponction par rapport à l'organe. Ce mode d'utilisation peut être adjoint au précédent. Le suivi temps réel cohérent avec la position de l'organe est assuré par l'algorithme de recalage; il peut néanmoins s'appuyer sur une information de localisation fournie par un localisateur externe (optique, magnétique, etc.). On parlera ci-après de « guidage per-opératoire » pour ce mode de réalisation.
- au temps post-opératoire, le système propose au clinicien une visualisation en 3D des ponctions effectuées, avec optionnellement, le cas échéant, une comparaison quantitative avec une précédente séance, dans un souci de qualité. L'information post-opératoire de distribution des prélèvements de tissu permet, en combinaison avec les résultats de l'analyse anatomopathologique, d'indiquer précisément les zones tumorales par rapport au volume prostatique. Cette information utile au diagnostic peut être partagée et transmise au thérapeute, qui aura la possibilité de focaliser le traitement.

Le système est constitué de différents éléments, comme illustré sur la figure 1.

Une sonde rectale (1) permet de positionner avec précision une aiguille de carottage (12).

Une aiguille de carottage (12) est en effet solidaire de la sonde rectale (1). Elle est susceptible d'occuper deux positions : une première position où ladite aiguille (12) est située à l'intérieur d'un guide solidaire de ladite sonde rectale (1) lorsque aucun tir de carottage n'est en cours, et une deuxième position où ladite aiguille (12) est située à l'extérieur de ladite sonde rectale (1) lorsqu'un tir de carottage est en cours afin de prélever du tissu biologique.

Une tête échographique (11) est disposée à l'extrémité de la sonde rectale, de manière à pouvoir observer sur un moniteur (3), via un système d'acquisition et d'analyse d'images (2), la position de ladite sonde rectale (1) par rapport à la prostate (non visible) lors de la manipulation de ladite sonde rectale (1). La sonde peut également être constituée par une sonde 3D à vision latérale ou oblique.

Ladite tête échographique (11) est une tête échographique 3D. Dans un mode de réalisation particulier, ladite tête échographique est une tête échographique à imagerie de type « 2,5 dimensions ». Ladite imagerie de type « 2,5 dimensions » est définie comme une imagerie mettant en oeuvre des images 2D spatialement localisées.

Dans une variante avantageuse, la tête échographique (11) est équipée d'un marqueur optique ou magnétique, apte à communiquer avec un localisateur optique ou magnétique, afin de faciliter la poursuite (en anglais « tracking ») temps réel per-opératoire.

Un moniteur (3) permet la visualisation du flux d'images acquises par la tête échographique (11). La visualisation du flux fonctionne à la cadence de rafraîchissement du système d'acquisition et d'analyse d'images (2). Ledit moniteur (3), ou dans une variante particulière un deuxième moniteur, permet simultanément la visualisation du référentiel « image de référence » ou du référentiel « image de suivi » qui peut comprendre les trajectoires de ponction planifiées, la trajectoire actuellement visée et les trajectoires effectuées antérieurement. La cadence de rafraîchissement de ladite visualisation du référentiel « image de référence » ou du référentiel « image de suivi » dépend des performances du système d'acquisition et d'analyse d'images (2). Ladite visualisation permet ainsi au clinicien d'observer la position de la sonde rectale (1) par rapport à l'organe et/ou d'observer la zone de prélèvement effectivement ponctionnée après déclenchement d'un tir de carottage.

Une table de commande (4) permet au clinicien d'effectuer les différents réglages nécessaires à la bonne exécution du schéma de biopsie. En particulier, il est possible de commander l'établissement d'un planning de biopsie, de choisir la manière d'identifier les cibles de prélèvements (de manière systématique, par calcul différentiel, à partir d'une image externe, ou manuellement), et de choisir la finesse des calculs lors du traitement des images.

De manière avantageuse, ledit moniteur (3) est un écran tactile affichant des objets graphiques virtuels correspondant à une partie au moins de ladite table de commande (4), ou de tout autre table de commande.

Un pistolet (13) permet d'effectuer des tirs de carottage. Il est en effet pourvu d'un actionneur (non visible) et relié mécaniquement à l'aiguille de carottage (12), de sorte que le déclenchement de l'actionneur par le clinicien provoque la demande d'acquisition d'une image par la tête échographique (11) à la prochaine période du procédé d'imagerie. Dans une variante avantageuse, la tête échographique (11) et le pistolet (13) sont en mode déclencheur (en anglais « trigger ») sans contact, pour l'acquisition automatique des images échographiques au moment du carottage.

Un réceptacle (non visible) est prévu pour le recueil des échantillons de tissu prélevés, avec un identifiant corrélé avec le marquage de la zone de prélèvement correspondant sur l'image unique de la prostate marquée.

Un dispositif de marquage, ou d'étiquetage, des prélèvements est utilisé afin de pouvoir identifier de manière univoque chaque zone de prélèvement. Les moyens d'étiquetage mis en oeuvre sont générés dans l'ordre chronologique des tirs de biopsie réalisés, quelque soit ledit ordre chronologique choisi par le clinicien, sans que cela n'altère la cohérence des résultats. En effet, le système n'est pas sensible à un décalage entre la répartition spatiale et l'ordre chronologique des tirs de biopsie, puisque à chaque prélèvement est associée une image qu'il n'est pas possible de confondre avec l'image associée à un autre prélèvement.

Dans un mode de réalisation particulier, les moyens d'étiquetage sont générés manuellement, aiguille de carottage en place.

Dans un autre mode de réalisation particulier, les moyens d'étiquetage univoque sont générés par un déclenchement automatique de l'acquisition de données par la capture d'un signal sonore, lumineux, magnétique ou tactile, au moment précis du tir de biopsie.

Le système d'acquisition et d'analyse d'images (2) est un système apte à communiquer, via des voies de communication (21, 22, 23), en outre avec la tête échographique (11) pour la commande des acquisitions d'images, avec la table de commande (4) pour les réglages par le clinicien, et avec le moniteur (3) pour la visualisation des images traitées.

Ledit système d'acquisition et d'analyse d'images (2) comprend un support informatique (non visible) permettant d'une part l'enregistrement des images acquises, et d'autre part l'enregistrement préalable d'un programme de commande d'un calculateur (non visible), pour la mise en oeuvre du procédé de traitement des images acquises et enregistrées, et du procédé d'estimation des positions des aiguilles de carottage par rapport à un planning de biopsie.

Ledit système d'acquisition et d'analyse d'images (2) comprend en outre un système échographique (non visible) permettant l'acquisition d'images et leur transfert vers le support informatique suscité.

Le procédé de traitement des images acquises et enregistrées met en oeuvre, en outre, un algorithme de recalage d'images basé sur l'optimisation d'une mesure de similarité, en partant d'une optimisation globale à partir de positions fournies par un modèle cinématique, et suivie d'une ou plusieurs optimisations locales. Une étape de précalcul des positions du modèle et des images correspondantes permet d'accélérer décisivement l'optimisation globale.

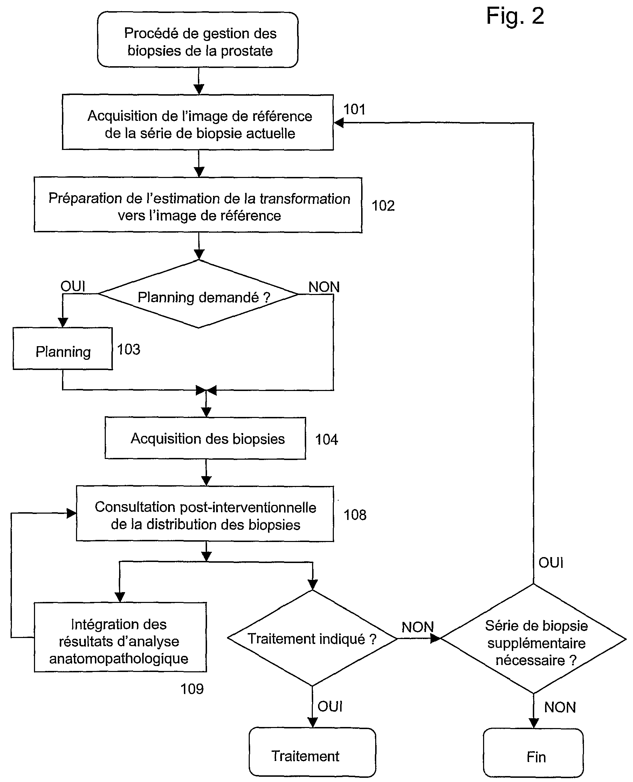

### La procédure de gestion des biopsies est illustrée par la figure 2.

Cette procédure présente principalement deux étapes :
- une séquence préliminaire comprenant l'acquisition de l'image de référence (101), la préparation de l'estimation de la transformation vers l'image de référence (étape 102) et éventuellement la préparation du planning de prélèvements (étape 103)
- une séquence de réalisation de la collection de prélèvements.

Ces étapes sont consécutives, le patient restant sur la table d'intervention, et les différentes séquences étant réalisées pendant la même intervention, sans déplacement significatif du patient, pendant une séance de biopsie unique, dont la durée est de quelques dizaines de minutes.

Le temps qui s'écoule entre l'acquisition de l'image de référence et la réalisation des prélèvements est de quelques secondes à quelques minutes.

La séquence préliminaire commence par l'acquisition de l'image de référence de la série de biopsie actuelle (101). Ladite image de référence est définie comme l'image entière de la prostate, acquise par le système d'acquisition et d'analyse d'images (2) au temps préliminaire, préalablement à la manipulation du système par le clinicien.

Cette étape d'acquisition de l'image de référence consiste à acquérir une ou plusieurs images échographiques adjacentes (par exemple trois images dans l'exemple décrit), avec la sonde d'échographie, le praticien modifiant l'orientation de la tête de la sonde entre chaque acquisition pour obtenir trois images distinctes de la prostate. Ces trois images sont fusionnées, par application d'un traitement sur ces trois images numériques pour recalculer une image unique, dite « image de référence » correspondant à une vue tridimensionnelle de la totalité de la prostate. Chacune des images initiales couvre généralement une partie seulement de la prostate, et la combinaison des images prises sous des angles légèrement différents permet de reconstruire une image de la totalité de la prostate, dans l'état, le positionnement et la configuration de la prostate et du patient correspondant à la séquence d'acquisition précédant d'un très bref laps de temps la séquence de prélèvement. Cette « image de référence » est également acquise avec les mêmes équipements, et dans les mêmes conditions que les images qui seront ensuite acquises pendant les étapes de prélèvement.

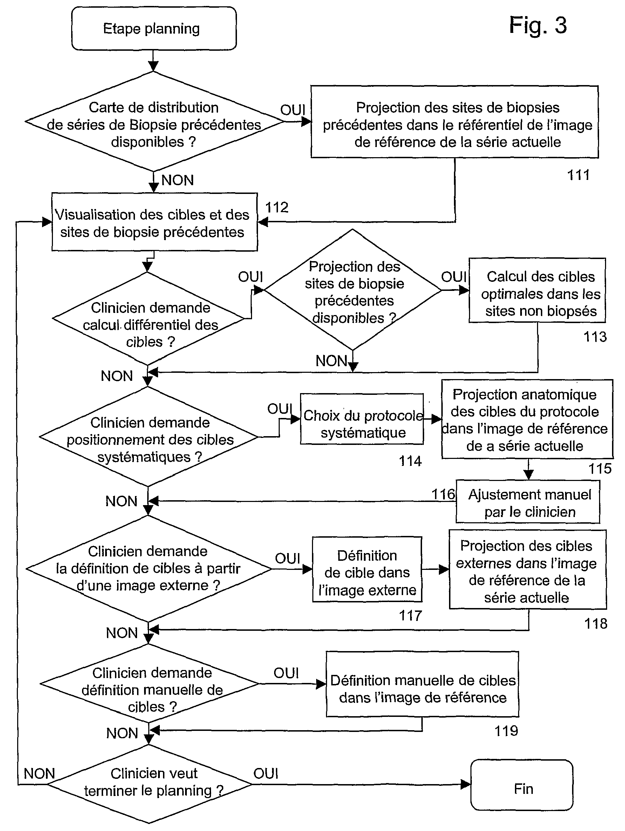

Cette image de référence sert à préparer les positions successives de la sonde rectale (1) sur la base du schéma de biopsie. À partir de ladite image de référence, le calculateur procède à une étape de préparation de l'estimation de la transformation vers l'image de référence (102). Cette étape de précalcul permet de pré-estimer les positions probables de la sonde et des images correspondantes à ces positions. Elle fait intervenir elle-même plusieurs étapes qui seront décrites ci-après et illustrées par la figure 7. Le clinicien dispose alors du choix entre l'établissement d'un planning (103) ou non. Le planning permet de disposer d'une carte de distribution d'une ou plusieurs séries de biopsies, facilitant son travail lors de l'acquisition des biopsies (104). L'étape d'établissement dudit planning sera décrite ci-après est illustrée par la figure 3. À partir du guidage de la sonde échographique (1), les images des différentes zones de prélèvement correspondant à l'éventuel planning (103) de biopsie sont acquises et stockées sur le support informatique du système d'acquisition et d'analyse d'images (2). Le résultat de l'acquisition des biopsies (104) ayant été obtenu, le praticien peut procéder à une consultation post-interventionnelle de la distribution des biopsies (108), en combinaison avec l'intégration des résultats d'analyse anatomopathologique (109), afin d'établir si un traitement est indiqué ou si une nouvelle série de biopsie supplémentaire est nécessaire, ou bien si le patient ne souffre assurément d'aucune pathologie au niveau de la prostate.

La procédure d'établissement du planning (103) de biopsie est illustrée par la figure 3. Si des cartes de distribution d'une ou plusieurs séries de biopsies précédentes du même patient sont disponibles, le calculateur réalise une projection des sites de biopsies précédentes dans le référentiel de l'image de référence de la série actuelle (111). Le praticien peut accéder à une visualisation des cibles et des sites de biopsie précédentes (112) via l'écran de visualisation (3). Le clinicien possède à ce niveau la possibilité de choisir l'établissement du planning de biopsie selon quatre modes distincts. Si le clinicien demande un calcul différentiel des cibles de prélèvement optimales, et si des projections des sites de biopsie précédents sont disponibles, le calculateur procède au calcul des cibles optimales dans les sites non biopsiés (113). Si le clinicien demande le positionnement de cibles systématiques à N prélèvements, N étant un paramètre défini à l'avance par ledit clinicien, le calculateur procède au choix du protocole systématique (114), puis à la projection anatomique des cibles dans l'image de référence de la série actuelle (115), l'ajustement manuel (116) étant ensuite réalisé manuellement par le clinicien. Si le clinicien demande la définition de cibles à partir d'une image de modalité autre que l'échographie (image externe obtenue par exemple par IRM), le calculateur procède à la définition de cible dans l'image externe (117), puis à la projection des cibles externes dans l'image de référence de la série actuelle (118), par mise en correspondance multi-modale. Enfin, si le clinicien demande la définition manuelle de cibles, c'est à lui de procéder à ladite définition manuelle de cibles dans l'image de référence (119). En dernier lieu, si le clinicien considère qu'il n'a pas terminé le planning, l'étape suivante consiste à nouveau à visualiser des cibles et des sites de biopsie précédente (112), avec à nouveau les mêmes choix à la disponibilité du clinicien. Sinon l'étape d'établissement du planning (103) de biopsie est terminée.

L'étape d'acquisition des biopsies (104) peut être réalisée en trois variantes, en fonction des capacités du système échographique et du dispositif informatique utilisés. La première variante dite de « guidage per-opératoire » (105) requiert un flux temps réel d'images échographiques 3D permettant d'identifier et de visualiser à tout moment la position de la trajectoire de ponction actuelle. Cette variante permet le guidage de la sonde dans la mesure où il y a acquisition et rafraîchissement de l'image à une cadence supérieure à 3 images par seconde. Le guidage de la sonde par le clinicien est par conséquent facilité, et par là le repérage en temps réel du prochain site de ponction. Par contre, l'acquisition de l'image n'étant pas réalisée au moment précis où un tir de biopsie est déclenché, l'image acquise et enregistrée ne correspond pas exactement au prélèvement effectué au moment du tir. Avantageusement, un système de détection du tir de biopsie, sonore, lumineux, magnétique ou tactile, peut aider à la synchronisation de l'image de suivi sur le tir. Dans la deuxième variante dite de « consultation per-opératoire » (106), l'acquisition et le transfert d'une image 3D se fait seulement après acquisition de la biopsie, enlevant ainsi la nécessité d'un flux temps réel, mais fournissant pourtant un retour per-opératoire immédiat sur la trajectoire de ponction. Si le système informatique le permet, ladite première variante et ladite deuxième variante peuvent être adjointes. Enfin, la troisième variante dite de « consultation post-opératoire » (107) se contente de l'enregistrement des images 3D en temps per-opératoire et ne procède à l'estimation des trajectoires de ponction qu'après l'intervention.

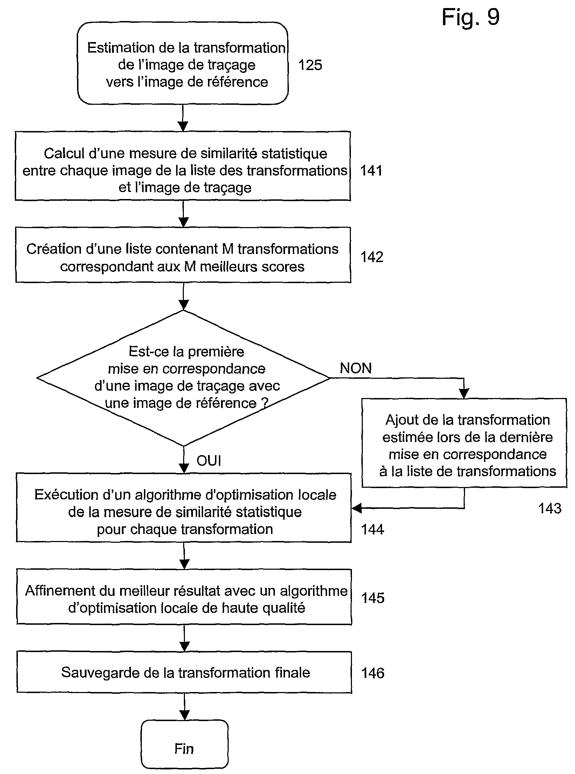

Dans la première variante, dite de « guidage per-opératoire » (105), et illustrée par la figure 4, l'acquisition de l'image de suivi (122) est réalisée indépendamment d'un tir du pistolet. Ladite image de suivi est définie comme l'image acquise par le système d'acquisition et d'analyse d'images au cours de la manipulation du système par le clinicien. Le système peut représenter en temps réel sur l'image échographique de guidage ou sur un modèle, la position actuelle du guide de ponction par rapport à l'organe. Le suivi temps réel cohérent avec la position de l'organe est assuré par l'algorithme de recalage de la présente invention ; il peut néanmoins s'appuyer sur une information de localisation fournie par un localisateur externe optique, magnétique, ou mécanique ou robotique. Si toutes les biopsies sont effectuées, cette procédure est terminée. Si toutes les biopsies n'ont pas été effectuées, un procédé constitué par une succession d'étapes (121 à 125) est réalisé, de manière à actualiser les données du dispositif et à acquérir une nouvelle image, à une cadence d'au moins 3 images par seconde. La première étape consiste en la mise à jour du rendu visuel de l'image de référence (121), comprenant les trajectoires de biopsie effectuées, les trajectoires de biopsie planifiées et l'estimation de la trajectoire de prélèvement. Elle permet au clinicien d'observer sur l'écran de visualisation (3) l'image de référence avec les informations nécessaires au guidage de la sonde rectale (1). Le procédé met ensuite en oeuvre une succession d'étapes (122 à 125), de manière à effectuer l'acquisition et le traitement de l'image. Cette succession d'étape comprend l'acquisition de l'image de suivi (122) par le dispositif d'imagerie (2). Le calculateur procède alors à l'estimation de la trajectoire de prélèvement dans la première image de la liste (123). Puis le procédé met en oeuvre l'estimation de la transformation de l'image de suivi vers l'image de référence (124). Cette estimation met en oeuvre des algorithmes de calcul et sera mieux illustrée par la figure 9. Le calculateur projette enfin la trajectoire estimée dans l'image de référence (125). Si lors de cette succession d'étapes, aucun tir n'a été effectué avec le pistolet, et si toutes les biopsies n'ont pas été effectuées, la procédure répète les étapes 121 à 125. Si par contre un tir a été effectué avec le pistolet durant le laps de temps entre deux rafraîchissements, alors le dispositif d'imagerie (2) procède au rajout de la trajectoire estimée aux trajectoires de biopsie effectuées (126). Si au regard de cette nouvelle trajectoire rajoutée toutes les biopsies ont été effectuées, la procédure est terminée. Sinon les étapes 121 à 125 sont répétées.

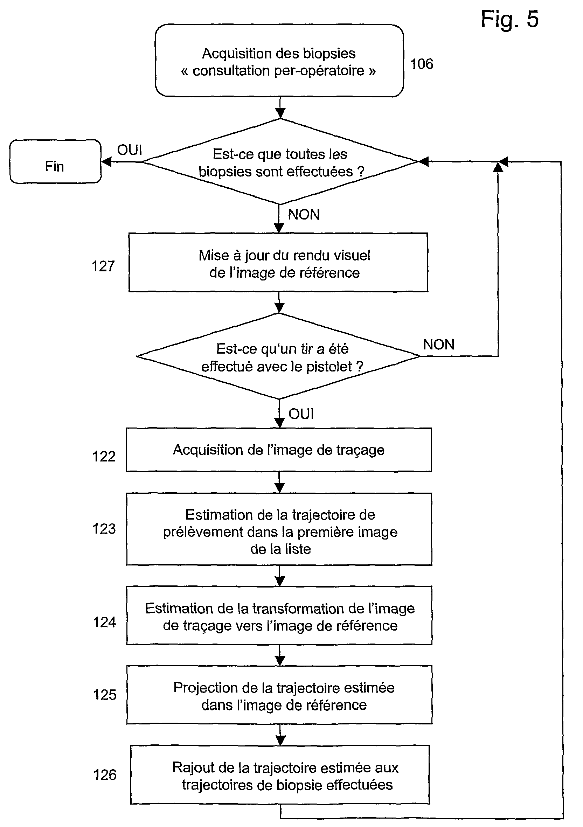

Dans la deuxième variante, dite de « consultation per-opératoire » (106), et illustrée par la figure 5, l'acquisition de l'image de suivi (122) est déclenchée par un tir du pistolet. Le système peut après chaque ponction représenter sur une vue annexe à l'image échographique temps réel, la position des prélèvements effectués jusque-là au cours de l'intervention. Si toutes les biopsies sont effectuées, cette procédure est terminée. Si toutes les biopsies n'ont pas été effectuées, la première étape consiste en la mise à jour du rendu visuel de l'image de référence (127). Cette étape comprend les trajectoires de biopsie effectuées et les trajectoires de biopsie planifiées. Tant qu'un tir de pistolet n'est pas déclenché, le système ne réalise que l'étape 121. Lorsqu'un tir de pistolet est déclenché, le procédé met en oeuvre une succession d'étapes (122 à 126), de manière à effectuer l'acquisition et l'enregistrement de l'image. Ces étapes 122 à 126 sont identiques aux étapes 122 à 126 décrites dans la première variante dite de « guidage per-opératoire » (figure 4). Si au regard de la nouvelle trajectoire rajoutée toutes les biopsies ont été effectuées, la procédure est terminée. Sinon l'étape 121 est répétée en boucle tant qu'un nouveau tir de pistolet n'est pas déclenché.

À noter que dans cette deuxième variante, l'utilisateur peut commander une image de suivi préalablement au tir de biopsie, afin de visualiser une estimation fiable de l'endroit courant visé par le pistolet.

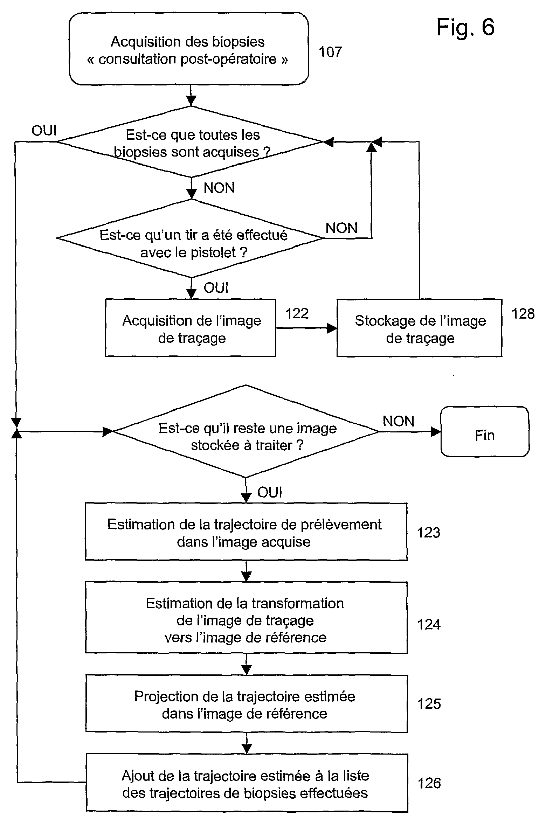

Dans la troisième variante dite de « consultation post-opératoire » (107), et illustrée par la figure 6, le traitement se fait au temps post-opératoire. Tant que toutes les biopsies ne sont pas acquises, à chaque tir effectué avec le pistolet le procédé met en oeuvre une étape d'acquisition de l'image de suivi (122) puis une étape de stockage de ladite image de suivi acquise (128). Si par contre toutes les biopsies ont été acquises, et tant qu'il reste au moins une image stockée à traiter, les étapes 123, 124, 125 et 126 telles que définies pour la procédure dite de « guidage per-opératoire » sont mises en oeuvre afin de traiter les images stockées.

Le procédé de préparation de l'estimation de la transformation vers l'image de référence (102), mis en oeuvre avant l'établissement éventuel d'un planning (103), est illustré par la figure 7. La première étape consiste à déterminer un volume englobant de la prostate (131). La position de la sonde est ensuite étalonnée relativement à l'image de référence (132). Le modèle cinématique est initialisé (133). Puis le calculateur procède au précalcul des positions probables de la sonde lors de la biopsie (134), puis au précalcul à partir de l'image de référence des images correspondantes aux positions de sonde précalculées (135). Plus exactement, pour chaque position fournie par le modèle cinématique, l'image de référence est ré-échantillonnée de telle manière que l'image résultante correspond à l'image de référence si elle avait été acquise sous cette position, dans la limite de l'information disponible dans l'image de référence et sans considération de déformations. Les images sont gardées en mémoire vive afin de permettre un accès rapide par la suite. Enfin, les images calculées et les transformations correspondantes sont stockées dans une liste (136). Cette liste d'éléments précalculés est utilisée lors de l'intervention par le procédé d'estimation de la transformation de l'image de suivi vers l'image de référence (125), illustré par la figure 9.

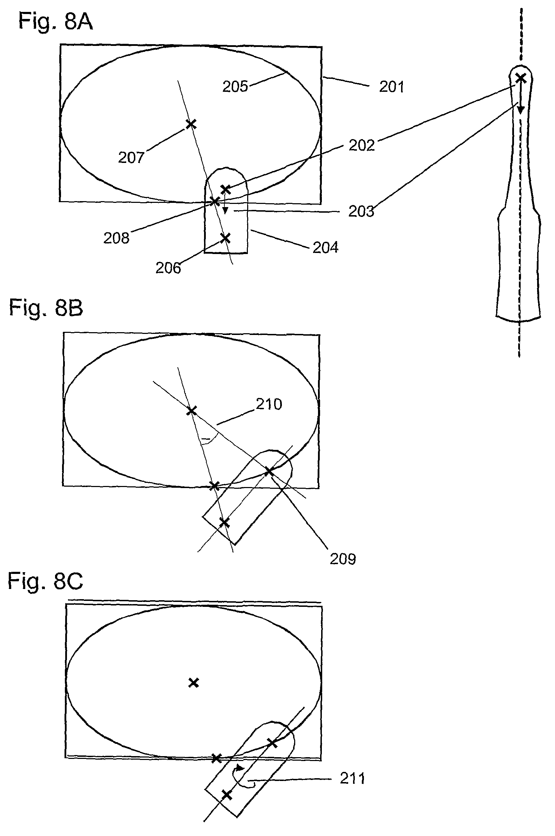

Le modèle cinématique est illustré par les figures 8A, 8B et 8C. Il est initialisé (133) avec une approximation de la surface de la prostate dans l'image de référence (205) et un point de rotation qui approxime les contraintes rotationnelles de la sonde dans le rectum (206). Il est également nécessaire de connaître la position de la tête échographique relative à l'image qu'elle produit, représenté par exemple par son centre (202), et l'orientation de la sonde, toujours relative à l'image, dite « axe de sonde » (203), qui peut être représentée par un vecteur pointant de la tête en direction de la manche de la sonde. Avec ces informations, il est possible d'estimer la position du rectum par rapport à la prostate et donc de la région sur la surface de la prostate qui peut être atteinte par la tête de la sonde. L'ensemble des positions de la sonde pour lesquelles la tête se trouve dans cette région et pour lesquelles l'axe de la sonde passe par le point de rotation fixe, est une bonne approximation de l'ensemble des positions de la sonde physiquement possible lors du positionnement de l'aiguille pendant une biopsie de la prostate. L'approximation peut être affinée en modélisant des fluctuations de pression exercée sur la prostate par le clinicien, en faisant varier la distance de la tête de la surface de quelques millimètres. Finalement il suffit de discrétiser le plus uniformément possible l'ensemble de positions décrites ci-dessus. À titre d'exemple, ce procédé peut être implémenté concrètement avec une approximation de la surface prostatique par un ellipsoïde (205) défini à partir d'un parallélépipède placé par le clinicien autour de la prostate visible dans l'image de référence (201). La région qui peut être atteinte par la sonde dans le rectum est estimée à partir du point d'intersection (208) de la ligne entre la tête de la sonde (202) et le centre de la surface (207) avec la surface, en passant par une représentation polaire de l'ellipsoïde (210), paramétrée par les angles alpha et bêta (210) tel que le point d'intersection coïncide avec la représentation pour alpha et bêta égaux à zéro. En bornant les valeurs absolues d'alpha et de bêta, une région est définie qui représente une approximation de la partie de la surface qui peut être atteinte par la tête de la sonde. L'ensemble des positions de la sonde pour lesquelles la tête se trouve sur la surface et l'axe passe par le point de rotation (206) peut être échantillonné en parcourant avec des pas discrets les paramètres alpha, bêta, chaque couple donnant un point de contact (210), et un troisième angle gamma (211) qui modélise les rotations de la sonde autour de son axe (203).

La figure 9 illustre le procédé d'estimation de la transformation de l'image de suivi vers l'image de référence (125), mise en oeuvre lors de l'acquisition des biopsies (104). À partir de l'estimation de la trajectoire de prélèvement dans la première image de la liste, ce procédé permet de projeter la trajectoire estimée dans l'image de référence. Tout d'abord, une mesure de similarité statistique est calculée entre chaque image de la liste des transformations et l'image de suivi (141). De manière avantageuse, le procédé fonctionne de manière avec une mesure de similarité du type « corrélation de Pearson », « information mutuelle normalisée », « somme des distances au carré », ou « rapport de corrélation ». De manière encore plus avantageuse, il est possible de les combiner ou de combiner les résultats de leur application sur plusieurs aspects de l'image, telle que sa dérivée ou sa laplacienne. Une liste est ensuite créée (142), ladite liste contenant M transformations correspondant aux M meilleurs mesures de l'étape 141. Une bonne robustesse du système est obtenue avec M=5. Si la mise en correspondance est la première mise en correspondance d'une image de suivi avec l'image de référence, le procédé passe à l'étape 144. Si la mise en correspondance n'est pas la première mise en correspondance d'une image de suivi avec l'image de référence, alors la transformation estimée lors de la dernière mise en correspondance est ajoutée à la liste des M transformations (143) avant de passer à l'étape 144. Ensuite, pour chaque transformation de la liste, un algorithme d'optimisation locale de la mesure de similarité statistique à partir de ladite transformation est mis en oeuvre (144). L'objectif de l'optimisation locale (144) est de trouver la transformation qui optimise la similarité dans un voisinage de la transformation initiale, en utilisant un algorithme d'optimisation locale classique tel que l'algorithme de Powell-Brent. Ensuite, le meilleur résultat issu de l'étape 144 est affiné avec un algorithme d'optimisation locale de haute qualité (145). Lors de cette étape, le même calcul que celui utilisé lors de l'étape 144 est appliqué, mais avec des paramètres permettant d'obtenir une précision plus fine au coût d'un temps de calcul plus long. Enfin, la transformation finale, donnant le meilleur résultat de similarité statistique, et affiné selon ledit algorithme de haute qualité, est sauvegardée (146).

La présente invention offre au praticien une meilleure prise en charge du cancer, et potentiellement un diagnostic plus rapide et une thérapie plus focalisée. De plus, les traitements médicaux et chirurgicaux, ainsi que les convalescences, peuvent être écourtés.

Également, le système a un grand intérêt dans l'apprentissage du geste. Il permet de montrer à un jeune praticien qu'il n'a pas effectivement ponctionné là où il l'attendait. D'où une accélération de sa courbe d'apprentissage.

L'échographie endorectale de prostate étant utilisée également pour un certain nombre de gestes thérapeutiques, souvent transpérinaux (curiethérapie, photodynamothérapie, cryothérapie, etc.), la présente invention peut s'appliquer également au temps thérapeutique. L'intérêt est alors de donner au temps per-opératoire au chirurgien ou radiothérapeute une information de localisation des aiguilles thérapeutiques au fur et à mesure qu'elles sont insérées, dans le respect d'un planning pré-opératoire.

Enfin, couplé à l'intégration d'informations anatomiques (régionalisation de la glande) ou statistiques (zones probables de présence du cancer), ou d'images de modalités complémentaires (IRM, Doppler, élastographie, image de contraste, CT, PET, fluorescence, etc.), ou encore des résultats de précédentes interventions sur le même patient, le système peut superposer ces nouvelles informations au volume échographique de référence, améliorant de fait le planning et le guidage du geste.

La mise en oeuvre du procédé d'imagerie peut avantageusement être assistée par un logiciel guidant le praticien. Ce logiciel guide le praticien dans la succession d'opérations à réaliser, de manière linéaire.

Ce logiciel va commander dans un premier temps l'affichage des informations relatives à la séquence préliminaire d'acquisition de l'image de référence.

Il commande les moyens de saisie des informations patient, ainsi le cas échéant des informations relatives aux conditions opératoires (date, nom de l'opérateur, informations pré-opératoires telles que PSA ou volume de la prostate, lieu de l'intervention, etc.)

Une fois que la totalité des informations obligatoires est enregistrée, le logiciel commande les fonctionnalités relatives à l'acquisition des images échographiques destinées au calcul de l'image de référence.

Lorsque la qualité de l'image de référence est validée par l'opérateur ou par un traitement automatique, le logiciel commande la séquence suivante correspondant au prélèvement des biopsies. Au cours de cette séquence, le logiciel permet d'enregistrer l'image échographique acquise au moment du déclenchement d'un nouveau prélèvement, et visualise en temps réel une image de regroupement faisant apparaître en superposition l'image de référence et la trajectoire du nouveau prélèvement, après une transformation de normalisation.

Lorsque la totalité des biopsies a été réalisée, l'utilisateur commande la préparation du rapport et l'archivage des données.

Le traitement de transformation des images acquises pour permettre une superposition avec l'image de référence peut être une transformation dans un espace cartésien, la prostate étant alors assimilée à un objet solide ne subissant aucune déformation pendant les étapes de prélèvement.

Il est possible d'améliorer le traitement par une transformation non-linéaire, prenant en compte la déformation de la prostate entre l'état au repos, et l'état de la prostate soumise à la pression locale de la tête de la sonde échographique, ainsi qu'à l'action de l'aiguille de prélèvement.

Ce traitement consiste à recalculer une image intermédiaire de la prostate lors d'un prélèvement par minimisation des écarts entre une zone ou des points de repère de l'image de référence et une zone de l'image acquise, pour faire correspondre au mieux une zone partielle ou des points de repère prédéterminés. Cette image intermédiaire correspond à un changement de repère de l'image acquise pour optimiser la superposition avec l'image de référence.

On procède ensuite à un deuxième traitement consistant à déformer l'image acquise et ainsi repositionnée, afin de faire correspondre d'autres zones ou points de référence de l'image acquise avec l'image de référence.

Cette deuxième transformation permet d'améliorer la qualité de la superposition, et donc de la représentation finale de la superposition des trajectoires de biopsie et de l'image de référence.

Afin d'améliorer encore la qualité de cette superposition, une solution avantageuse consiste à prévoir une étape de validation de l'image acquise recalculée. Cette validation peut être réalisée manuellement, par l'opérateur, vérifiant si l'image recalculée se superpose, lors de sa visualisation, d'une façon pertinente sur l'image de référence. Cette vérification est réalisée avec une visualisation tri-dimensionnelle permettant à l'opérateur d'explorer dans l'espace la superposition d'images et procéder à des vérifications sous différentes orientations.

Cette vérification peut être automatisée par un traitement d'évaluation des décalages entre l'image de référence et l'image recalculée par le calcul d'une mesure de similarité permettant de valider automatiquement lorsque le résultat de cette mesure est inférieur à une valeur seuil.

La mesure de similarité est évaluée à chaque pas au cours de la phase de convergence de la méthode de recalage. La fonction de mesure calcule, selon un exemple de mise en oeuvre, les écarts entre des structures canoniques repérées dans l'image de référence et l'image acquise, pour délivrer en fin de parcours une évaluation des écarts finaux, donc de la qualité de la superposition.

Une autre variante consiste à intégrer d'autres images acquises préalablement ou ultérieurement pour les superposer avec l'image de référence et les trajectoires de biopsies. Ces autres images sont par exemple une image IRM de la prostate, une image issue d'un modèle anatomique ou un modèle de répartition statistique des zones de cancer, ou encore une série d'images acquises sur le même patient à des dates différentes.

## Revendications

1. Système pour l'imagerie prostatique, comprenant une sonde rectale (1) équipée d'un guide de ponction actif (12,13) et un calculateur (2), la sonde comportant une tête d'échographie (11) pour la réalisation d'une image de la zone de ponction effectuée à l'aide d'une aiguille (12) actionnée par l'opérateur, le calculateur (2) comprenant:
- des moyens pour l'enregistrement et le traitement d'une image de référence liée à la position initiale de la prostate à partir d'images échographiques acquises par ladite tête d'échographie ;
- des moyens pour l'enregistrement et le traitement des images acquises par ladite tête d'échographie agencés de sorte à calculer des transformations des images acquises par ladite tête d'échographie vers un référentiel « image de référence » lié à la position initiale de la prostate; et,
- des moyens d'enregistrement d'une partie au moins des images acquises comprenant la localisation des différentes positions de l'aiguille en vue d'une visualisation (3) de leurs représentations sur une image unique comprenant une partie au moins de la prostate,
**caractérisé en ce que** les moyens pour l'enregistrement et le traitement des images acquises par ladite tête d'échographie sont agencés de sorte à calculer les transformations des images acquises vers le référentiel « image de référence » par un algorithme de recalage d'image basé sur une optimisation locale d'une mesure de similarité.

2. Système pour l'imagerie prostatique selon la revendication 1, **caractérisé en ce qu'**il comporte en outre un réceptacle pour le recueil des échantillons de tissu prélevés, avec un identifiant corrélé avec le marquage de la zone de prélèvement correspondant sur l'image unique de la prostate marquée.

3. Système pour l'imagerie prostatique selon l'une au moins des revendications 1 à 2, **caractérisé en ce que** la sonde rectale est une sonde à main sans positionnement robotisé.

4. Système pour l'imagerie prostatique selon l'une au moins des revendications 1 à 2, **caractérisé en ce que** la sonde rectale est une sonde à main naviguée par un localisateur optique ou magnétique.

5. Système pour l'imagerie prostatique selon l'une au moins des revendications 1 à 2, **caractérisé en ce que** la sonde rectale est une sonde à positionnement robotisé.

6. Système pour l'imagerie prostatique selon l'une au moins des revendications 1 à 5, **caractérisé en ce qu'**il comporte des moyens de traitement des images acquises enregistrées comprenant la localisation des différentes positions de l'aiguille en vue d'une visualisation de la position de l'aiguille de prélèvement dans le référentiel « image de référence » ou un référentiel « image de suivi », en surimpression avec une image d'une partie au moins de la prostate présentant les différentes positions de prélèvements antérieurs.

7. Système pour l'imagerie prostatique selon la revendication 6, **caractérisé en ce qu'**il comporte des moyens de traitement des images acquises enregistrées comprenant la localisation des différentes positions de l'aiguille en vue d'une visualisation en temps réel de la position de l'aiguille de prélèvement dans le référentiel « image de référence » ou le référentiel « image de suivi », en surimpression avec une image d'une partie au moins de la prostate présentant les différentes positions de prélèvements antérieurs.

8. Système pour l'imagerie prostatique selon l'une au moins des revendications 6 à 7, **caractérisé en ce que** les moyens de traitement des images acquises enregistrées sont agencés de sorte à permettre une visualisation de la distribution cancéreuse dans le référentiel « image de référence » après fusion de la distribution spatiale des ponctions marquées avec les résultats issus de l'analyse anatomopathologique.

9. Système pour l'imagerie prostatique selon l'une au moins des revendications 1 à 8, **caractérisé en ce qu'**il comporte des moyens de recalage d'une nouvelle image prostatique marquée lors de chaque nouveau prélèvement.

10. Système pour l'imagerie prostatique selon l'une au moins des revendications 1 à 9, **caractérisé en ce que** la fréquence de rafraîchissement des images acquises par la sonde échographique est d'au moins 3 images par seconde.

11. Système pour l'imagerie prostatique selon l'une au moins des revendications 1 à 10, **caractérisé en ce que** la sonde échographique est agencée de sorte à procéder à une imagerie tridimensionnelle.

12. Système pour l'imagerie prostatique selon l'une au moins des revendications 1 à 11, **caractérisé en ce que** la sonde échographique est agencée de sorte à procéder à une imagerie de type « 2,5 dimensions ».

13. Système pour l'imagerie prostatique selon l'une au moins des revendications 1 à 12, **caractérisé en ce qu'**il comporte des moyens d'étiquetage univoque de chaque zone de prélèvement par un marquage chronologique avec un numéro d'ordre.

14. Système pour l'imagerie prostatique selon la revendication 13, **caractérisé en ce que** les moyens d'étiquetage univoque sont mis en oeuvre par un déclenchement automatique de l'acquisition de données par la capture d'un signal sonore, lumineux, tactile ou magnétique, au moment précis du tir de biopsie.

15. Système pour l'imagerie prostatique selon l'une au moins des revendications 1 à 14, **caractérisé en ce que** les moyens pour l'enregistrement et le traitement des images acquises mettent en oeuvre, en outre, un modèle cinématique dérivé des contraintes anatomiques spécifiques d'une ponction transrectale de la prostate, permettant d'identifier des positions probables de la sonde lors de l'intervention.

16. Système pour l'imagerie prostatique selon l'une au moins des revendications 1 à 15, **caractérisé en ce que** les moyens pour l'enregistrement et le traitement des images acquises mettent en oeuvre, en outre, un processus de précalcul des positions probables de la sonde lors de la biopsie et des images correspondant aux positions de la sonde précalculées à partir de l'image de référence.

17. Système pour l'imagerie prostatique selon l'une au moins des revendications 1 à 16, **caractérisé en ce qu'**il comporte des moyens d'assistance par un logiciel commandant successivement la saisie des informations patient, l'affichage des informations relatives à la séquence préliminaire d'acquisition de l'image de référence, les fonctionnalités relatives à l'acquisition des images échographiques destinées au calcul de l'image de référence et lorsque la qualité de l'image de référence est validée par l'opérateur ou par un traitement automatique, la séquence de prélèvement des biopsies.

18. Système pour l'imagerie prostatique selon l'une au moins des revendications 1 à 17, **caractérisé en ce que** les moyens pour l'enregistrement et le traitement des images mettent en oeuvre une transformation non-linéaire, prenant en compte la déformation de la prostate entre l'état au repos, et l'état de la prostate soumise à une pression.

19. Système pour l'imagerie prostatique selon l'une au moins des revendications 1 à 18, **caractérisé en ce qu'**il comporte des moyens de validation de l'image acquise recalculée.

20. Système pour l'imagerie prostatique selon la revendication 19, **caractérisé en ce que** les moyens de validation évaluent automatiquement des décalages entre l'image de référence et l'image recalculée.

21. Système pour l'imagerie prostatique selon la revendication 20, **caractérisé en ce que** les moyens de validation évaluent des écarts entre des structures canoniques repérées dans l'image de référence et l'image acquise, pour délivrer une évaluation des écarts finaux.

22. Système pour l'imagerie prostatique selon l'une au moins des revendications 1 à 21, **caractérisé en ce qu'**il comporte des moyens de traitement des images acquises enregistrées agencés de sorte à permettre une intégration d'autres images acquises préalablement ou ultérieurement pour les superposer avec l'image de référence et les trajectoires de biopsies.

## Patentansprüche

1. System zur Prostata-Bildgebung, das eine Rektalsonde (1) umfasst, die mit einem aktiven Punktionsführer (12, 13) und einem Rechner (2) ausgestattet ist, wobei die Sonde einen Ultraschallkopf (11) zum Herstellen eines Bilds der Zone der Punktion, die mit Hilfe einer Nadel (12) ausgeführt wird, die von dem Bediener betätigt wird, umfasst, wobei der Rechner (2) Folgendes umfasst:
- Mittel zum Aufzeichnen und zur Verarbeitung eines Referenzbilds, das mit der anfänglichen Position der Prostata verbunden ist, ausgehend von Ultraschallbildern, die von dem Ultraschallkopf erfasst werden,
- Mittel zum Aufzeichnen und zur Behandlung der von dem Ultraschallkopf erfassten Bilder, die derart eingerichtet sind, dass Umformungen der Bilder, die von dem Ultraschallkopf erfasst werden, zu einem "Referenzbild"-Referenzwerk, das mit der anfänglichen Position der Prostata verbunden ist, berechnet werden, und
- Mittel zum Aufzeichnen mindestens eines Teils der erfassten Bilder, die die Lage der unterschiedlichen Positionen der Nadel umfassen, zur Anzeige (3) ihrer Darstellungen auf einem einzigen Bild, das mindestens einen Teil der Prostata umfasst,
**dadurch gekennzeichnet, dass** die Aufzeichnungs- und Verarbeitungsmittel der Bilder, die von dem Ultraschallkopf erfasst werden, derart eingerichtet sind, dass die Umformungen der erfassten Bilder zu dem "Referenzbild"-Referenzwerk durch einen Bild-Neueinstellungsalgorithmus, der auf einer lokalen Optimierung einer Ähnlichkeitsmessung basiert, berechnet werden.

2. System zur Prostata-Bildgebung nach Anspruch 1, **dadurch gekennzeichnet, dass** es außerdem einen Behälter zum Aufnehmen entnommener Gewebeproben mit einem Identifikator, der mit der Kennzeichnung der entsprechenden Entnahmezone auf dem einzigen markierten Bild der Prostata korreliert ist, umfasst.

3. System zur Prostata-Bildgebung nach mindestens einem der Ansprüche 1 bis 2, **dadurch gekennzeichnet, dass** die Rektalsonde eine manuelle Sonde ohne robotisierte Positionierung ist.

4. System zur Prostata-Bildgebung nach mindestens einem der Ansprüche 1 bis 2, **dadurch gekennzeichnet, dass** die Rektalsonde eine manuelle Sonde ist, die von einem optischen oder magnetischen Lokalisator geführt wird.

5. System zur Prostata-Bildgebung nach mindestens einem der Ansprüche 1 bis 2, **dadurch gekennzeichnet, dass** die Rektalsonde eine Sonde mit robotisierter Positionierung ist.

6. System zur Prostata-Bildgebung nach mindestens einem der Ansprüche 1 bis 5, **dadurch gekennzeichnet, dass** es Mittel zur Verarbeitung der erfassten aufgezeichneten Bilder umfasst, die die Lokalisierung der unterschiedlichen Positionen der Nadel für eine Anzeige der Position der Entnahmenadel in dem "Referenz Bild"-Referenzwerk oder "Mitverfolgungsbild"-Referenzwerk in Überlagerung mit einem Bild mindestens eines Teils der Prostata, das die unterschiedlichen Positionen früherer Entnahmen präsentiert, umfassen.

7. System zur Prostata-Bildgebung nach Anspruch 6, **dadurch gekennzeichnet, dass** es Mittel zur Verarbeitung der erfassten aufgezeichneten Bilder umfasst, die die Lokalisierung der unterschiedlichen Positionen der Nadel für eine Anzeige in Echtzeit der Position der Entnahmenadel in dem "Referenzbild"-Referenzwerk oder dem "Mitverfolgungsbild"-Referenzwerk in Überlagerung mit einem Bild mindestens eines Teils der Prostata, das die unterschiedlichen Positionen früherer Entnahmen präsentiert, umfassen.

8. System zur Prostata-Bildgebung nach mindestens einem der Ansprüche 6 bis 7, **dadurch gekennzeichnet, dass** die Mittel zur Verarbeitung der erfassten aufgezeichneten Bilder derart eingerichtet sind, dass sie eine Anzeige der Krebsverteilung in dem "Referenzbild"-Referenzwerk nach Fusion der räumlichen Verteilung der Punktionen, die mit den Resultaten markiert sind, die aus der anatomopathologischen Analyse hervorgehen, erlauben.

9. System zur Prostata-Bildgebung nach mindestens einem der Ansprüche 1 bis 8, **dadurch gekennzeichnet, dass** es Mittel zum Neueinstellen eines neuen Prostatabilds, das bei jeder neuen Entnahme markiert wird, umfasst.

10. System zur Prostata-Bildgebung nach mindestens einem der Ansprüche 1 bis 9, **dadurch gekennzeichnet, dass** die Auffrischfrequenz der Bilder, die von der Ultraschallsonde erfasst werden, mindestens 3 Bilder pro Sekunde beträgt.

11. System zur Prostata-Bildgebung nach mindestens einem der Ansprüche 1 bis 10, **dadurch gekennzeichnet, dass** die Ultraschallsonde derart eingerichtet ist, dass eine dreidimensionale Bildgebung vorgenommen wird.

12. System zur Prostata-Bildgebung nach mindestens einem der Ansprüche 1 bis 11, **dadurch gekennzeichnet, dass** die Ultraschallsonde derart eingerichtet ist, dass eine Bildgebung des "2,5-dimensionalen" Typs vorgenommen wird.

13. System zur Prostata-Bildgebung nach mindestens einem der Ansprüche 1 bis 12, **dadurch gekennzeichnet, dass** es Mittel zur eindeutigen Etikettierung jeder Entnahmezone durch eine chronologische Markierung mit einer laufenden Nummer umfasst.

14. System zur Prostata-Bildgebung nach Anspruch 13, **dadurch gekennzeichnet, dass** die Mittel zum eindeutigen Etikettieren durch ein automatisches Auslösen der Datenerfassung durch Erfassen eines akustischen, Leucht-, taktilen oder magnetischen Signals genau im Augenblick der Biopsieauslösung umgesetzt werden.

15. System zur Prostata-Bildgebung nach mindestens einem der Ansprüche 1 bis 14, **dadurch gekennzeichnet, dass** die Mittel zum Aufzeichnen und zur Verarbeitung der erfassten Bilder außerdem ein kinematisches Modell umsetzen, das von den spezifischen anatomischen Gegebenheiten einer transrektalen Punktion der Prostata abgeleitet ist, das es erlaubt, die wahrscheinlichen Positionen der Sonde bei dem Eingriff zu identifizieren.

16. System zur Prostata-Bildgebung nach mindestens einem der Ansprüche 1 bis 15, **dadurch gekennzeichnet, dass** die Mittel zum Aufzeichnen und zur Verarbeitung der erfassten Bilder außerdem einen Vorab-Berechnungsprozess der wahrscheinlichen Positionen der Sonde bei der Biopsie und der Bilder, die den ausgehend von dem Referenzbild vorab berechneten Positionen entsprechen, umsetzen.

17. System zur Prostata-Bildgebung nach mindestens einem der Ansprüche 1 bis 16, **dadurch gekennzeichnet, dass** es Mittel zur Unterstützung durch eine Software umfasst, die nacheinander die Erfassung der Patienteninformationen, das Anzeigen der Informationen in Zusammenhang mit der Vorabsequenz zur Erfassung des Referenzbilds, die Funktionalitäten in Zusammenhang mit der Erfassung der Ultraschallbilder, die zur Berechnung des Referenzbilds bestimmt sind, und, wenn die Qualität des Referenzbilds vom Bediener oder durch eine automatische Verarbeitung bestätigt wird, die Entnahmesequenz der Biopsien steuert.

18. System zur Prostata-Bildgebung nach mindestens einem der Ansprüche 1 bis 17, **dadurch gekennzeichnet, dass** die Mittel zum Aufzeichnen und zur Verarbeitung der Bilder eine nicht lineare Umformung umsetzen, die die Verformung der Prostata zwischen dem Ruhezustand und dem Zustand der Prostata, die einem Druck ausgesetzt ist, berücksichtigt.

19. System zur Prostata-Bildgebung nach mindestens einem der Ansprüche 1 bis 18, **dadurch gekennzeichnet, dass** es Mittel zum Bestätigen des neu berechneten erfassten Bilds umfasst.

20. System zur Prostata-Bildgebung nach Anspruch 19, **dadurch gekennzeichnet, dass** die Bestätigungsmittel automatisch Verlagerungen zwischen dem Referenzbild und dem neu berechneten Bild beurteilen.

21. System zur Prostata-Bildgebung nach Anspruch 20, **dadurch gekennzeichnet, dass** die Bestätigungsmittel Abweichungen zwischen kanonischen Strukturen, die in dem Referenzbild und dem erfassten Bild gekennzeichnet sind, beurteilen, um eine Beurteilung der abschließenden Abweichungen zu liefern.

22. System zur Prostata-Bildgebung nach mindestens einem der Ansprüche 1 bis 21, **dadurch gekennzeichnet, dass** es Mittel zur Verarbeitung der aufgezeichneten erfassten Bilder umfasst, die derart eingerichtet sind, dass sie eine Integration anderer früher oder später erfasster Bilder erlauben, um sie mit dem Referenzbild und den Biopsiebahnen zu überlagern.

## Claims

1. Prostate imaging system, comprising a rectal probe (1) equipped with an active biopsy guide (12, 13) and a computer (2), the probe including an ultrasound head (11) for producing an image of the biopsy area performed using a needle (12) actuated by the operator, the computer (2) comprising:
- means for recording and processing a reference image linked with the initial position of the prostate based on ultrasound images acquired by said ultrasound head;
- means for recording and processing images acquired by said ultrasound head arranged so as to compute conversions of the images acquired by said ultrasound head to a "reference image" repository linked with the initial position of the prostate; and,
- means for recording at least part of the images acquired comprising the localisation of the different positions of the needle with a view to a visualisation (3) of the representations thereof on a single image comprising at least part of the prostate,
**characterised in that** the means for recording and processing the images acquired by said ultrasound head are arranged so as to compute the conversions of the images acquired to the "reference image" repository by an image repositioning algorithm based on a local optimisation of a similarity measurement.

2. Prostate imaging system according to claim 1, **characterised in that** it further includes a receptacle for collecting the tissue samples removed, with an identifier correlated with the tagging of the corresponding sampling area on the tagged single image of the prostate.

3. Prostate imaging system according at least one of claims 1 to 2, **characterised in that** the rectal probe is a manual probe without robotic positioning.

4. Prostate imaging system according to at least one of claims 1 to 2, **characterised in that** the rectal probe is a manual probe navigated by an optical or magnetic localiser.

5. Prostate imaging system according at least one of claims 1 to 2, **characterised in that** the rectal probe is a robotic positioning probe.

6. Prostate imaging system according to at least one of claims 1 to 5, **characterised in that** it includes means for processing the recorded acquired images comprising the localisation of the different positions of the needle with a view to a visualisation of the position of the sampling needle in the "reference image" repository or a "follow-up image" repository, overlaid with an image of at least part of the prostate having the different prior sampling positions.

7. Prostate imaging system according to claim 6, **characterised in that** it includes means for processing the recorded acquired images comprising the localisation of the different positions of the needle with a view to a real-time visualisation of the position of the sampling needle in the "reference image" repository or the "follow-up image" repository, overlaid with an image of at least part of the prostate having the different prior sampling positions.

8. Prostate imaging system according to at least one of claims 6 to 7, **characterised in that** the means for processing the recorded acquired images are arranged so as to enable a visualisation of the cancerous distribution in the "reference image" repository after merging the spatial distribution of the tagged biopsies with the results of the anatomopathological analysis.

9. Prostate imaging system according to at least one of claims 1 to 8, **characterised in that** it includes means for repositioning a new prostate image tagged during each new sampling.

10. Prostate imaging system according to at least one of claims 1 to 9, **characterised in that** the refresh frequency of the images acquired by the ultrasound probe is at least 3 images per second.

11. Prostate imaging system according to at least one of claims 1 to 10, **characterised in that** the ultrasound probe is arranged so as to perform three-dimensional imaging.

12. Prostate imaging system according to at least one of claims 1 to 11, **characterised in that** the ultrasound probe is arranged so as to perform "2.5-dimensional" type imaging.

13. Prostate imaging system according to at least one of claims 1 to 12, **characterised in that** it includes means for univocal tagging of each sampling area by chronological tagging with a sequential number.

14. Prostate imaging system according to claim 13, **characterised in that** the univocal tagging means are implemented by automatic activation of data acquisition by the capture of an audio, light, touch or magnetic signal, at the exact biopsy launch time.

15. Prostate imaging system according to at least one of claims 1 to 14, **characterised in that** the means for recording and processing the images acquired further use a kinematic model derived from the specific anatomic constraints of a transrectal prostate biopsy, suitable for identifying the probable positions of the probe during the procedure.

16. Prostate imaging system according to at least one of claims 1 to 15, **characterised in that** the means for recording and processing the images acquired further use a process for pre-computing the probable positions of the probe during the biopsy and the images corresponding to the positions of the probe pre-computed on the basis of the reference image.

17. Prostate imaging system according to at least one of claims 1 to 16, **characterised in that** it comprises software assistance means successively controlling the input of patient information, the display of the information relating to the preliminary acquisition sequence of the reference image, the functions relating to the acquisition of the ultrasound images intended for the computation of the reference image and when the quality of the reference image is validated by the operator or by automatic processing, the biopsy sampling sequence.

18. Prostate imaging system according to at least one of claims 1 to 17, **characterised in that** the means for recording and processing the images use a nonlinear conversion, accounting for the deformation of the prostate between the resting state, and the state of prostate subject to pressure.

19. Prostate imaging system according to at least one of claims 1 to 18, **characterised in that** it includes means for validating the recomputed acquired image.

20. Prostate imaging system according to claim 19, **characterised in that** the validation means automatically evaluate offsets between the reference image and the recomputed image.

21. Prostate imaging system according to claim 20, **characterised in that** the validation means evaluate deviations between canonical structures identified in the reference image and the acquired image, in order to issue an evaluation of the final deviations.

22. Prostate imaging system according to at least one of claims 1 to 21, **characterised in that** includes means for processing the recorded acquired images arrange so as to enable an integration of further images acquired previously or subsequently in order to overlay same with the reference image and the biopsy trajectories.
